(19) Europäisches Patentamt
European Patent Office
Office européen des brevets

(11) **EP 1 916 987 B1**

(12) **EUROPÄISCHE PATENTSCHRIFT**

(45) Veröffentlichungstag und Bekanntmachung des Hinweises auf die Patenterteilung:
**16.06.2010 Patentblatt 2010/24**

(21) Anmeldenummer: **06775894.6**

(22) Anmeldetag: **23.08.2006**

(51) Int Cl.:
*A61K 8/19* (2006.01)      *A61K 8/36* (2006.01)
*A61Q 11/00* (2006.01)      *A61K 9/02* (2006.01)
*A61K 9/20* (2006.01)

(86) Internationale Anmeldenummer:
**PCT/DE2006/001475**

(87) Internationale Veröffentlichungsnummer:
**WO 2007/022765 (01.03.2007 Gazette 2007/09)**

(54) **NAHRUNGSMITTELZUSATZ ZUR REMINERALISIERUNG VON ZAHNSCHMELZ**

FOOD ADDITIVE FOR REMINERALISATION OF TOOTH ENAMEL

COMPLEMENT ALIMENTAIRE POUR REMINERALISATION DE L'EMAIL DENTAIRE

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK RS**

(30) Priorität: **25.08.2005 DE 102005040423**

(43) Veröffentlichungstag der Anmeldung:
**07.05.2008 Patentblatt 2008/19**

(73) Patentinhaber: **MEDERER Süßwarenvertriebs GmbH**
**90765 Fürth (DE)**

(72) Erfinder: **LAHRSOW, Jobst**
**97941 Tauberbischofsheim (DE)**

(74) Vertreter: **Rau, Manfred**
**Rau, Schneck & Hübner**
**Patentanwälte**
**Königstraße 2**
**90402 Nürnberg (DE)**

(56) Entgegenhaltungen:
**EP-A1- 0 673 913      WO-A-00/44245**
**GB-A- 1 298 299      GB-A- 2 341 798**
**US-A- 5 851 578**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung bezieht sich auf die Verwendung eines Nahrungsmittelzusatzes als konzentriertes Additiv zur Remineralisation von Zahnschmelz. Die Erfindung bezieht sich ferner auf eine Süßigkeit mit einem derartigen Nahrungsmittelzusatz.

Für den menschlichen Körper sind Mineralstoffe existenziell nötige, nicht organische Nährstoffe. Weil der Organismus sie nicht selbst produzieren kann, müssen sie über die Nahrung zugeführt werden. Ebenso wie Vitamine sind die Minerale jedoch keine Energieträger, d. h. sie sind am Energiestoffwechsel praktisch nicht beteiligt.

Die meisten Mineralien sind sogenannte Bau- oder Regler-Stoffe. Zu den Baustoffen gehören Calcium, Phosphor und Magnesium und zu den Regler-Stoffen Jod, Natrium, Kalium, Eisen und Chlor. Wenige Mineralien weisen beide Eigenschaften zugleich auf. So ist zum Beispiel Phosphor am Aufbau von Knochen und Zähnen und zugleich an der Regulation des Säure-Basen-Haushaltes beteiligt.

Im menschlichen Organismus sind Mineralstoffe für viele Funktionen ein unerlässlicher Bestandteil. Zum Aufbau von Körpersubstanzen wie Knochen, Zähnen und Muskeln geben die Mineralstoffe Festigkeit und Belastbarkeit. Wesentliche, notwendige Eigenschaften von Körperflüssigkeiten werden durch gelöste Mineralstoffe als Elektrolyte beeinflusst. Dazu zählt zum Beispiel die Aufrechterhaltung des osmotischen Druckes.

Mineralstoffe sind auch wesentliche Bestandteile von organischen Verbindungen im Körper. Jod ist Bestandteil des Schilddrüsenhormons, Kobalt ist in Vitamin B 12 enthalten, der Blutfarbstoff Hämoglobin benötigt Eisen. Gemäß den Empfehlungen der Deutschen Gesellschaft für Ernährung benötigen Jugendliche und Erwachsene je nach Alter und Geschlecht wenigstens 1000 bis 1200 mg Calcium pro Tag, 700 bis 1250 mg Phosphor, 350 bis 400 mg Magnesium, 12 bis 15 mg Eisen, 150 bis 200 mg Jod, 7 bis 10 mg Zink und andere Mineralstoffe, sogenannte Spurenelemente, in kleineren Mengen. Der menschliche Organismus hat sich im Laufe seiner Entwicklung über Tausende von Generationen hinweg auf eine Nahrung mit wenigstens 2/3 pflanzlichem Bestandteil eingestellt. Eine aktuelle Untersuchung von K. Gedrich und G. Karg an der TU München ergab, dass die tatsächlich Ernährung in Deutschland von einer in Bezug auf ausreichende Mineralstoffversorgung optimierten Ernährung drastisch abweicht: Im Vergleich zur Sollmenge ist die tatsächliche Verzehrmenge für Obst und Gemüse auf die Hälfte abgesunken, für Getreideprodukte und Kartoffel auf 2/3. Bei Frauen ist die Gemüseverzehrmenge sogar auf 1/3 abgesunken, der Verzehr von Fleisch, Fisch und Eiern hingegen auf das 1,3-fache angestiegen. An deren Stelle sind Nahrungsmittel getreten, die durch küchentechnische und industrielle Verfahren zubereitet sind. Diese Verfahren bewirken einen zum Teil erheblichen Verlust an Mineralstoffen und Spurenelemente. Daraus ergibt sich eine drastische Reduzierung der durchschnittlichen Versorgung der Bevölkerung mit Mineralstoffen.

Gemäß dem Stand der aktuellen Technik gibt es zahlreiche Zusätze für Nahrungsmittel, welche dem vorbeschriebenen Mangel abhelfen sollen. Sie weisen jedoch verschiedene Nachteile auf. Gemäß Patent DE 103 49 050 A1 soll bindungsfähiges Calcium und Phosphat in Lebensmittel wie Fruchtgummi, Gelastineprodukte oder Süßwaren eingebracht werden. Nachteilig ist, dass bei Einbringung der richtliniengemäßen Menge an mineralstoffhaltigen Beimischungen diese während des Herstellungsprozesses als Kristalle ausfallen und das Produkt in unzulässiger Weise eintrüben. Wenn die Beimischungen auf das Maß reduziert werden, bei dem sich keine Kristalle mehr bilden, ist der Anteil an Mineralstoffen derart gering, dass die erwünschte Wirkung fast vernachlässigbar gering wird.

Bei vorgenanntem Verfahren lässt sich das Problem der unerwünschten Kristallisierung dadurch reduzieren, dass ein reaktiver Calciumspender aus definierten Verbindungen oder Mischungen angewandt wird, dem zusätzlich unterschiedlich stark calciumkomplexierende Säuren zugefügt werden können. Nachteilig ist jedoch, dass ein zu hoher Wasseranteil in der Lösung wiederum die erreichbare Konzentration an Calcium beschränkt und somit wiederum die Wirksamkeit auf ein unbedeutendes Maß reduziert.

Die WO 00/44245 offenbart ein Lebensmittelsalzprodukt, das eine oder mehrere Hydratformen von Magnesiumamoniumchlorid oder Calciumamoniumchlorid enthält. Die offenbarten Lebensmittelsalzprodukte sind kristallin.

Aus der EP 0 673 913 A1 ist die Herstellung und die Verwendung von Calcium-Alkalimetallcitrat-Verbindungen als Arzneimittel bekannt. Bei den offenbarten Verbindungen handelt es sich um kristalline Verbindungen.

Die GB 2 341 798 A offenbart eine nährstoffhaltige oder pharmazeutische Zusammensetzung, der eine oder mehrere wasserfreie Verbindungen zugemischt sind, um freiwerdendes Wasser zu binden. Durch das Zumischen der wasserfreien Verbindungen entsteht ein Trocknungseffekt.

Aus der US 5 851 578 ist ein Getränk bekannt, das eine verbesserte Löslichkeit für Calcium-Verbindungen aufweist. Das Calcium wird in Form eines wasserlöslichen Salzes in das Getränk eingebracht.

Die GB 1 298 299 A offenbart ein Nahrungsergänzungsmittel, das leicht dissoziierbare organische Salze von Natrium, Calcium, Kalium und Magnesium enthält, wobei das Atomverhältnis von Natrium zu Kalium in einem definierten Bereich liegt.

Ein alternativer Lösungsvorschlag wird von Jarcho im US Patent 4 097 935 beschrieben. Er schlägt eine übersättigte Lösung von Hydroxylapatit als Mundspüllösung vor. Auch hier ist eine erreichbare Konzentration so gering, dass nur ein extrem ausgedehntes und unrealistisch häufiges Spülen eine wunschgemäße Wirkung erzielen lässt.

Im US Patent 4 080 440 beschreibt Digiulio eine metastabile Lösung von Calcium und Phosphat bei niedrigem pH-Wert. Als Wirkmechanismus wird erwartet, dass nach Anstieg des pH-Wertes in den demineralisierten Poren des Zahnschmelzes Calcium und Phosphat ausfallen, insbesondere zusammen mit den katalytisch wirkenden Fluorionen. Hier besteht die Gefahr, dass bereits vor der beabsichtigten Wirkung der Zahnschmelz durch den niedrigen pH-Wert demineralisiert wird und das Gewebe Schaden nimmt.

Mit US-Patent 4,606,912 versucht Rudy die Anwendung einer wässrigen Lösung mit einer Calciumionenquelle sowie einem Chelatbildner für Calciumionen. Wegen der schwierigen Kontrolle des Chelatbildners ist dieses Verfahren jedoch unpraktikabel.

In verschiedenen Varianten schlägt Tung (US-Patente 5,037,639 und 5,268,167 und 5,437,857 sowie 5,460,803) ein Pulver vor, welches Calciumsalze, Phosphate und Carbonate enthält. Nach Auflösung im Speichel präzipitiert dieses Pulver ein amorphes Calciumphosphat. Problematisch ist jedoch dessen Beständigkeit.

Insgesamt zeigt das Beispiel der Remineralisation von Zahnschmelz, dass wesentliche Probleme von Zusatzstoffen zur Mineralstoffversorgung nicht gelöst werden konnten. Die drei wichtigsten Nachteile im besprochenen Beispiel sind,

dass die tatsächlich erreichte Konzentration von Calcium- und von Phosphationen für eine effektive Wirkung zu gering ist oder die Lösung zu wässrig und demzufolge weitgehend unwirksam ist, oder

die Lösung zu wässrig und demzufolge weitgehend unwirksam ist, oder

der stark vom physiologischen pH-Wert 4,5 abweichende pH-Wert die Schleimhäute von Mund, Rachen, Magen und Verdauungstrakt schädigt bzw. eine Einarbeitung in Nahrungsmittel sehr stark erschwert.

Der Erfindung liegt die Aufgabe zugrunde, die Remineralisation von Zahnschmelz mittels eines Nahrungsmittelzusatzes zu verbessern.

Diese Aufgabe wird durch eine Verwendung eines Nahrungsmittelzusatzes als konzentriertes Additiv zur Remineralisation von Zahnschmelz mit den Merkmalen des Anspruchs 1 gelöst. Ferner wird diese Aufgabe durch eine Süßigkeit, umfassend einen Nahrungsmittelzusatz als konzentriertes Additiv zur Remineralisation von Zahnschmelz mit den Merkmalen des Anspruchs 8 gelöst.

Bekannt ist, dass die Koordinationszahl die Anzahl der nächsten Nachbarionen um das Ion ist und die Hydratisierung die Anlagerung von $H_2O$ an das Ion ist.

Dieser Nahrungsmittelzusatz hat zahlreiche Vorteile. Nach diesem Prinzip sind fast alle Mineralstoffe und Spurenelemente, die der menschliche Organismus benötigt, zuführbar und zwar in einer ionisierten Form, so dass sie besonders leicht verwertbar sind. Für die Verarbeitung in der Nahrungsmittelherstellung sind die wichtigsten Vorteile, dass die Salzhydratschmelze in ihrer amorph erstarrten Form über längere Zeit gelagert werden kann ohne instabil zu werden, dass sie in ihrer Viskosität sehr einfach auf den Produktionsprozess des Nahrungsmittels eingestellt werden kann, indem entweder eine geringe Wassermenge hinzugefügt wird und/oder die Temperatur verändert wird. Dadurch, dass zwei Parameter zur Einstellung der Viskosität vorhanden sind, ist eine Anpassung an Prozesse mit gegebener Temperatur oder an Prozesse mit kritischer Viskosität problemlos möglich.

Für die Kombination eines erfindungsgemäßen Nahrungsmittelzusatzes mit anderen Nahrungsmitteln ist es vorteilhaft, dass eine Vielzahl von organischen Säuren wie Gluconsäure, Milchsäure. Zitronensäure, Essigsäure, Äpfelsäure, Fumarsäure, Valeriansäure, Ascorbinsäure, Cystein, Glutarsäure oder andere/weitere für Nahrungsmittel geeignete Säuerungsmittel sowie deren Salze bei der Herstellung verwendbar sind. Vorteilhaft ist auch, dass in einer erfindungsgemäßen Variante als Ionenquelle auch anorganische Säurereste, also z. B. Chloride, Sulfate, Phosphate, Fluoride, Carbonate oder deren partiell veresterte Abkömmlinge einsetzbar sind. Dabei ergibt sich als Vorteil, dass bei Salzen mit anorganischem Säurerest wie zum Beispiel Chloriden, der Säurerest ein niedriges Molekulargewicht aufweist. Daher kann im fertigen Produkt ein sehr hoher Gewichtsanteil an Kationen erreicht werden Durch den Einsatz von z. B. Chloriden lässt sich unter Beibehaltung der Viskosität der Anteil an Kationen im Endprodukt deutlich erhöhen.

Der maximale Gewichtsanteil der Kationen am Gesamtgewicht des fertigen Produktes wird im Wesentlichen durch das Molekulargewicht und die Wertigkeit der Anionen bestimmt. Je nach Zusammensetzung der Salzmischung ergibt sich ein unterschiedlicher, maximaler, theoretischer Kationenanteil. Wenn die drei Salze $CaA_1$, $CaA_2$ und $CaA_3$ mit den Molekulargewichten $M_1$, $M_2$ und $M_3$ in den molaren Anteilen $x_1$, $x_2$ und $x_3$ gemischt sind, wobei $x_1 + x_2 + x_3 = 1$ ist, ergibt sich als maximaler, molarer Gehalt an Ca:

$$Ca_{max.} = 1/(x_1 * M_1 + x_2 * M_2 + x_3 * M_3)$$

Das erfindungsgemäße Produkt kann mit einem Mineralstoffgehalt von 10 % bis 95 % des nach obiger Formel berechneten, maximal möglichen Mineralstoffgehaltes produziert und verarbeitet werden. Durch geringfügige Variation des Wassergehaltes kann die Viskosität in weiten Grenzen variiert werden.

Vorteilhaft ist, dass der gewünschte pH-Wert der Salzhydratschmelze durch Auswahl der Zusatzstoffe vorbestimmt werden kann. Für Lebensmittel im physiologischen Bereich um 6,5 bis 7 sind Schmelzen mit Salzen von anorganischen sowie organischen Säuren geeignet. Für Nahrungsmittel mit niedrigerem (saurem) pH-Wert können Salzhydratschmelzen mit einem Überschuss an Säuren hergestellt werden. Sie eignen sich als Zusatz für saure Speisen oder Süßigkeiten. Mit sauren Salzhydratschmelzen wird sogar die Beimischung von Mineralstoffen in die Zuckerschmelze von Hartkaramell möglich, auch Kaumassen aus Gelatine und anderen Verdickungsmitteln können durch den Zusatz von Mineralstoffen verbessert werden.

Beispiele für physiologisch neutrale Schmelzen sind: Calciumlactat und Calciumgluconat oder erweitert um Magnesiumlactat. Für die Calciumausstattung von sauren Schmelzen kann Calciumlactat mit Gluconsäure angesäuert werden. Je nach dem gewünschten Geschmack des Nahrungsmittel können auch Äpfelsäure oder Zitronensäure oder Äpfelsäure gemeinsam mit Zitronensäure sowie andere zugelassene Säuerungsmittel zugegeben werden. Für die Ausstattung mit Magnesium wird die Schmelze aus Gluconsäure und Calciumlactat angereichert mit Magnesiumsalzen, z. B. der Essigsäure, Gluconsäure, Milchsäure oder auch der Salzsäure. Ein praktisches Beispiel sind saure Kaumassen wie z. B. Fruchtgummis, bei denen die Salzhydratschmelze eine saure Mischung aus Calciumgluconat, -lactat, -malat sowie -citrat sein kann.

Ein weiterer Vorteil der erfindungsgemäßen Nahrungsmittelzusätze ist, dass die Geschmacksrichtung des Produktes gesteuert werden kann. Calciumlactate generieren einen schwach bitteren Geschmack und eignen sich damit für Erfrischungsgetränke, Mixgetränke, Puddings und Speisen mit Bittermandelgeschmack oder herbe Biersorten außerhalb des deutschen Reinheitsgebotes. Ein stark bitterer Geschmack wird durch Calciumchlorid oder Magnesiumchlorid bewirkt. Eine süßliche Geschmacksnote mit leicht bitterem Nachgeschmack wird mit einer Komponente von Calciumacetat erreicht; geeignet also z. B. in Orangenmarmeladen, Süßigkeiten und Fruchtsaftgetränken ähnlich Bitter Lemon. Für Anwendungsfälle, in denen eine Beeinflussung des Geschmackes nicht vorteilhaft ist, wird in der Mischung das geschmacksneutrale Magnesium oder Calciumgluconat vorherrschen. In Anwendungsfällen, bei denen die Geschmacksrichtung "bitter" entweder willkommen ist oder zu einem neuen Geschmacksgefühl komponiert werden kann oder durch geeignete Aromen überdeckt werden kann, bietet die erfindungsgerechte Ausführungsform mit Salzen der anorganischen Säure als Kationenquelle eine Lösung. Die Möglichkeit, z. B. die stark bitter schmeckenden Salze Magnesiumchlorid und Calciumchlorid zu verwenden, birgt den Vorteil, dass vergleichsweise sehr hohe Gewichtsanteile der Mineralstoffe beigemischt werden können. Dieses ist insbesondere für Calciumchlorid sehr vorteilhaft, da - wie zuvor erwähnt - Calcium derjenige Mineralstoff ist, den der menschliche Organismus in der größten Menge benötigt.

Im menschlichen Organismus eines erwachsenen Mannes sind rund 1 kg Calcium enthalten. Davon sind 99,9 % in Zähnen und Knochen eingelagert. Zu deren Aufbau und Erhalt wird laufend Calcium benötigt, wie vorerwähnt, je nach Geschlecht und Alter zwischen 1000 und 1200 mg pro Tag als Mindestwert.

In einer statistisch relevanten Untersuchung wurde ermittelt, dass die tägliche Calciumaufnahme bei 2/3 der Erwachsenen in Deutschland geringer ist als 800 mg pro Tag. Die Entkalkung der Knochen (Osteoporose) und der Zähne (Karies) ist auch darauf zurückzuführen.

Auch in den Muskeln kann der Mangel an Calcium bei intensiven sportlichen Betätigungen zu Zittern und Krämpfen führen. Im fortgeschrittenen Stadium tritt eine gesteigerte Erregbarkeit des Nervensystems auf, Verkrampfungen der Muskulatur (Tetanie) und Missempfindungen, wie zum Beispiel Kribbeln, Pelzigkeit oder Ameisenlaufen (Parästhesien) sind zu beklagen. An diesen Mangelerscheinungen wird deutlich, dass Calcium für die elektrischen Aktionspotentiale für Muskel und Nerven mit verantwortlich ist.

Auch im Blutkreislauf ist Calcium wichtig. Hier wirkt es als sogenannter Gerinnungsfaktor IV.

Mit einer ausreichenden Versorgung an Calciumionen sind die vorgenannten Mangelerscheinungen vermeidbar.

Eine Korrektur durch ein Nahrungsmitteladditiv ist auch deshalb von Vorteil, weil Probleme durch Überdosierung nicht bekannt sind. Der menschliche Stoffwechsel wird nur diejenige Menge an Calciumionen aufnehmen, die zu seiner Funktion erforderlich sind. Darüber hinaus vorhandene Calciumionen werden auf natürlichem Wege wieder ausgeschieden. Erst bei extremer Überdosierung zusammen mit einer genetischen Vordisposition ist in einigen wenigen Fällen eine Bildung von Nierensteinen beobachtet worden. In der Regel ist dieser Effekt jedoch mit einer extremen Überernährung verknüpft, die rechtzeitig erkannt und abgestellt werden kann.

Eine weitere vorteilhafte Wirkung von Calcium als Nahrungsmittelzusatz ist die sogenannte Remineralisation der Zähne: Nach dem Verzehr vergärbarer Kohlenhydrate bilden die Bakterien der Zahnbeläge verstärkt Säuren. Dadurch fällt der pH-Wert im Speichel in der Umgebung der Zähne ab.

Der Speichel ist im Normalfall, also bei pH-Werten zwischen pH 6 und pH 7 stets mit einer sogenannten Hydroxylapatit-Phase übersättigt. Dieses gelöste Material repariert Schmelzdefekte, wie initiale Demineralisationen oder Haarrisse.

Wenn durch Übersäuerung ein pH-Wert von 5,5 unterschritten wird, gerät der Speichel in die Untersättigung und dem Zahnschmelz wird Hydroxylapatit entzogen (Demineralisation). Dabei entstehen im Zahnschmelz Poren, die sich im fortgeschrittenen Stadium zur Karies auswachsen.

Sobald der pH-Wert des Speichels den Wert von 5,5 wieder überschreitet, wird im Speichel die Sättigungsgrenze an Hydroxylapatit wieder überschritten, so dass die durch die Säureattacke entstandenen Poren durch das im Speichel gelöste Mineral wieder aufgefüllt werden (Remineralisation).

Falls durch allzu häufigen Verzehr zuckerhaltiger Speisen und/oder wegen unzureichender Entfernung der Plaques die Demineralisationsphasen in ihrer Wirkung die Remineralisationsphasen überwiegen, wird laufend Mineral aus dem Schmelz ausgeschwemmt. Die Poren vertiefen sich und es entsteht eine Kariesläsion. Um diesem Ablauf Einhalt zu gebieten, ist eine zusätzliche Versorgung des Speichels mit Calciumionen während der Demineralisationszeit hilfreich. Durch Erhöhen der Calciumkonzentration im Speichel wird der Konzentrationsgradient zwischen diesem und den Plaques kleiner. Das hat zur Folge, dass weniger oder kein Calcium aus den Plaques in den Speichel diffundiert. Der Diffusionsstrom kann sogar umgekehrt werden und zur forcierten Remineralisation des Schmelzes beitragen.

Eine erhöhte Calciumkonzentration in den Plaques bringt weitere Vorteile. Aufgrund der typischen zeitlichen pH-Wert-Profile in den Zahnbelägen nach Verzehr zuckerhaltiger Kost kann bei ausreichender Calciumkonzentration unter der katalytischen Wirkung von Fluorid die Remineralisation des Zahnschmelzes am Grund der Pore beginnen. Bei zeitlich konstantem pH-Wert, beschränkt sich die Remineralisation, insbesondere bei erhöhter Fluoridkonzentration, im wesentlichen auf die äußerste 20 bis 100 $\mu$m dicke Oberflächenschicht. Dadurch kann es in ungünstigen Fällen zur Bildung eines Deckels über der Pore kommen.

Ein weiterer, erfindungsgemäßer Vorteil sind bisher nicht in dieser Breite transportierbare Mineralstoffmengen als mögliche Beimischungen für Zahnpasta, Mundwässer, andere Mundhygieneartikel, Lippenstifte, andere auf die Lippen aufzutragende Salben oder Flüssigkeiten oder oral oder rektal einzuführenden Arzneimittel und andere Pillen, Kapseln oder Zäpfchen. Auch hier werden neue Möglichkeiten der Beimischung dadurch eröffnet, dass die Viskosität des Nahrungsmittelzusatzes sehr einfach durch Zusatz von Wasser einstellbar ist, also etablierte und bewährte Herstellungsverfahren nicht oder nur unwesentlich geändert werden müssen. Die bekannte, den Mundgeruch verhindernde Wirkung des Zinks ließe sich einfach in Zahnpasten und Mundspüllösungen einführen. Außerdem ist die Lagerungsdauer der Salzhydratschmelze sehr hoch.

Aus dieser Stabilität folgen neue Möglichkeiten, um die menschlichen Nahrungsmittel und die menschliche Trinkwasser- und Getränkeversorgung mit Mineralstoffen zu versehen. Die Erfindung schlägt u. a. vor, die Innenseite von Trinkhalmen mit erfindungsgemäßen Salzhydratschmelzen auszustatten. Durch das vorbeifließende Getränk erfolgt ein kontinuierlicher Abbau der auf der Innenschicht angelagerten, mineralstoffhaltigen Salzhydratschmelze. Selbstverständlich wird der Trinkhalm dadurch zu einem Nahrungsmittel, welches entsprechend zu kennzeichnen und mit einem entsprechenden Verfallsdatum zu versehen ist.

In Weiterführung dieser Idee sind auch die Innenflächen von Trinkwasserspendern, Trinkwasseraufbereitungsgeräten oder Maschinen zur Herstellung von Eiswürfeln zu beschichten.

Weitere vorteilhafte Anwendungen sind die Beimischung zu Getränken aller Art. Das Spektrum beginnt bei Getränken mit einem möglichst niedrigen Kaloriengehalt, welche der Flüssigkeitsversorgung dienen mit der erwähnten Möglichkeit, zusätzlich zur Mineralstoffbeimischung auch gleichzeitig die Geschmacksnote einstellen zu können. Bei diesen Getränken führt der Zusatz von erfindungsgemäßen Nahrungsmittelzusätzen nicht zu einer unerwünschten Erhöhung des Kaloriengehaltes.

Am anderen Ende des möglichen Spektrums stehen kalorienhaltige Getränke wie Milch, Kakao, Wein oder Bier. Hier ist der erfindungsgerechte Nahrungsmittelzusatz dadurch interessant, dass er in einer geschmacksneutralen Variante das wesentliche Charakteristikum des Getränkes unangetastet lässt, jedoch mit dem zusätzlichen Werbeargument des erweiterten Mineralstoffgehaltes versehen ist.

Eine weitere erfindungsgemäße Fortsetzung dieses Gedankens ist, dass aus einem Vorratsbehälter über eine Rohrverbindung in den vorgenannten oder ähnlichen Maschinen eine mineralstoffhaltige Salzhydratschmelze dem Getränk oder dem Trinkwasser beigemischt wird.

Die konsequente Applikation dieses Prinzips eröffnet die Möglichkeit, öffentliche Trinkwasserversorgungen durch die gezielte Beimischung von erfindungsgemäßen Salzhydratschmelzen für die Versorgung der gesamten Bevölkerung mit Mineralstoffen einzusetzen.

Sehr interessant ist die Möglichkeit, bisher vom Image als wenig gesundheitsfördernd eingestufte Genussmittel wie Pralinen, Lakritz, Kuchen, Kekse, Marmelade., Pommes Frites und Hamburger durch erhöhten Mineralstoffgehaltes werblich deutlich aufwerten zu können.

Im Folgenden sollen weitere Einzelheiten und Merkmale der Erfindung anhand von Beispielen näher erläutert werden. Die abgebildeten Beispiele sollen die Erfindung jedoch nicht einschränken sondern nur erläutern. Es zeigen in schematischer Darstellung:

Figur 1    Tabellarische Darstellung von Koordination und Wechselwirkung im System Salz-Wasser

Figur 2    Remineralisation einer Pore im Zahnschmelz

Die Figuren zeigen im Einzelnen:

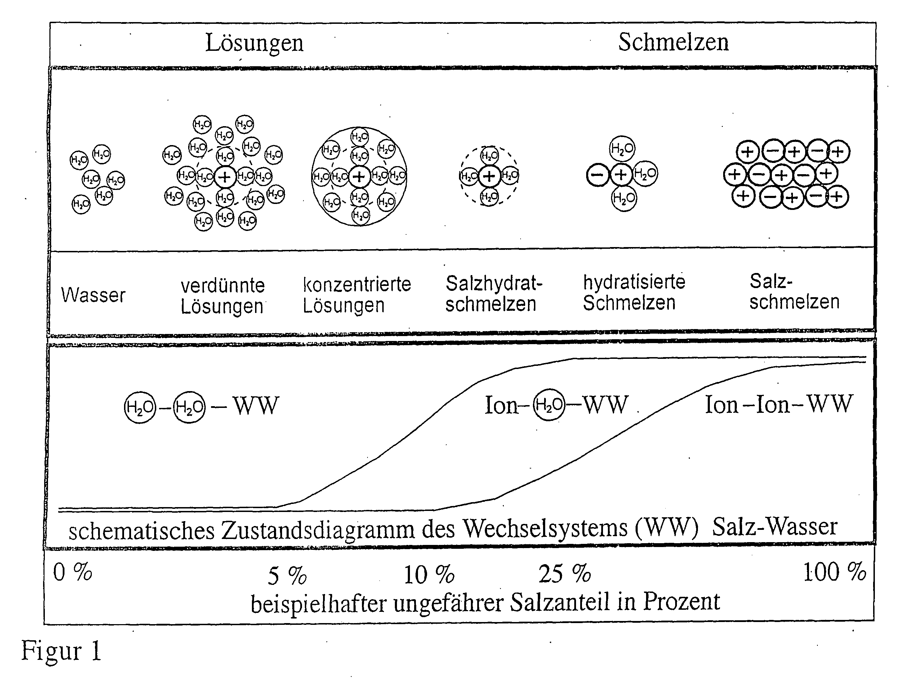

Figur 1 zeigt skizzenhaft die schematische Struktur von Salzhydratschmelzen in der Einordnung von reinem Wasser über verdünnte Salzlösungen bis zu hydratisierten Salzschmelzen.

Oben sind die Strukturen der molekularen Anordnung wiedergegeben. Dabei sind die Wassermoleküle durch $H_2O$, die Kationen durch einen Kreis mit einem Pluszeichen und die Anionen durch einen Kreis mit einem Minüszeichen dargestellt.

In der nächsten Zeile sind die Bezeichnungen niedergelegt, beginnend bei reinem Wasser über Lösungen und endend bei konzentrierten Salzschmelzen.

In der vierten Zeile sind drei verschiedene Wechselwirkungen benannt und ihr jeweiliger geschätzter Anteil an der Gesamtwechselwirkung.

In der letzten Zeile ist der jeweilige Salzanteil beziffert.

In den Spalten der Figur 1 sind vier charakteristische Stufen eines Salz-Wasser-Systems wiedergegeben:

An der linken Seite der Abbildung, ausgehend von reinem Wasser, ist die Struktur einer verdünnten Lösung mit einem Salzanteil bis zu 5% eingetragen. Hier ist die spezifische Wechselwirkung (WW) vor allem auf das Wasser ausgerichtet.

In der dritten Spalte der Tabelle ist die Struktur einer konzentrierten Salzlösung mit einem Salzanteil bis 10% wiedergegeben. Hier machen sich neben den Wasser-Wasser-Wechselwirkungen die Ion-Ion-Wechselwirkungen schon deutlich bemerkbar.

Als anderes Extrem beschreibt die ganz rechte Spalte der Tabelle einen Salzanteil von 100%. Wasser ist auch nicht mehr als Kristallwasser vorhanden. Es liegt eine reine Salzschmelze vor, bei der ausschließlich Wechselwirkungen zwischen den Salzionen auftreten

In der vierten Spalte von links wird eine Salzhydratschmelze charakterisiert: In der oberen Zeile ist als Struktur ein Kation vollständig von Wassermolekülen umgeben. Die Anzahl von vier Wassermolekülen steht als Beispiel für die Koordinationszahl vier des Kations.

Infolge der "dünneren" Hydrathülle nimmt die Abschirmung der Ionen durch die Wassermoleküle ab, was durch einen einzigen, gestrichelten Kreis in der Struktur symbolisiert wird. Damit kommt verstärkt wie Wechselwirkung zwischen den Ionen zum Tragen.

Im schematischen Zustandsdiagramm ist als hauptsächliche Welchselwirkung für eine Salzhydratschmelze die Wechselwirkung zwischen dem Ion und Wassermolekülen dargestellt. Zu erkennen ist (am unteren Rand der vierten Spalte), dass eine Salzhydratschmelze auch einen Anteil von Wechselwirkungen zwischen den Ionen aufweist.

Durch dieses Feld der Abbildung wird ein ganz wesentliches Merkmal von Salzhydratschmelzen beschrieben, nämlich das Auftreten von drei verschiedenen Arten der Wechselwirkung:

- Wechselwirkung zwischen den Wassermolekülen
- Wechselwirkung zwischen dem Ion und dieses umgebende Wassermoleküle
- Wechselwirkung zwischen den Ionen

Der Salzanteil einer Salzhydratschmelze ist im vorliegenden Beispiel mit 10 bis 25 % (Mol-%) benannt.

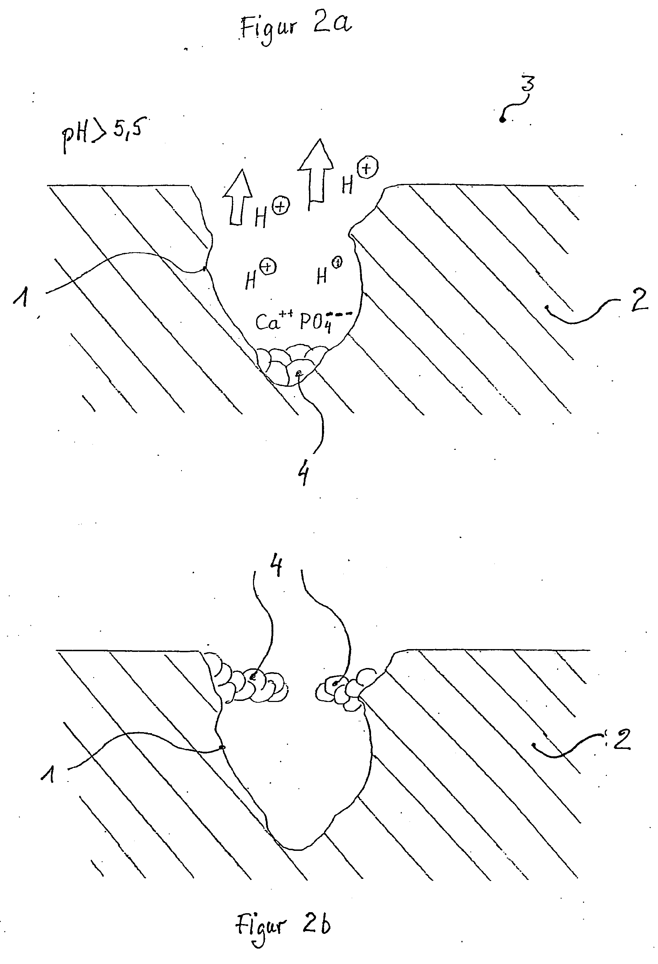

In Figur 2 sind 2 Querschnitte durch eine Pore 1 im Zahnschmelz 2 gezeichnet. Im oberen Beispiel, Figur 2a, ist im Speichel der Mundhöhle 3 der pH-Wert (durch Vergärung von Kohlenhydraten) über pH=5,5 hinaus abgefallen. Das dadurch entstehende Ungleichgewicht an Hydroxylapatit im Speichel 3 saugt Hydroxylapatit-Ionen an, in Figur 2a gekennzeichnet durch $H^+$. Der fehlende Hydroxylapatit würde mangels anderer Quellen dem Zahnschmelz 2 entzogen werden. In der gezeichneten Konfiguration mit zeitlich variierendem pH-Profil, verbunden mit einem Überangebot an Calciumphosphat in der Mundhöhle, wird unter der katalytischen Wirkung von Fluorid die Hydroxylapatit-Untersättigung vom Calciumphosphat im Speichel ausgeglichen. Die Figur 2 lässt erkennen, wie in den Tiefen der Pore Calciumphosphat angelagert und zu Hydroxylapatit-Kristallen 4 aufgebaut wird. Dieser Prozess sorgt für eine Remineralisation der Zähne.

Im Vergleich dazu zeigt Figur 2b die Remineralisation mit dem Katalysator Fluorid bei zeitlich schwach im Neutralbereich variierenden pH-Wert. Infolge der im Neutralbereich hohen Übersättigung des Speichels an Mineral läuft der Prozess der Kristallbildung vermehrt an der Schmelzoberfläche ab; denn, die abgeschiedene Menge ist proportional zur Abscheidungsdauer, während die Diffusionszeit mit dem Quadrat der Diffusionsstrecke zunimmt: Durch die Abscheidung auf dem Weg in den Zahn wird der Diffusionsstrom zu tieferen Schichten hin ausgedünnt. Die tieferen Schichten werden von weniger Mineral erreicht. Am Rand der Pore bilden sich Hydroxylapatitkristalle 4 als Deckel aus.

Im Folgenden sind einige Beispiele für die mögliche Zusammensetzung von Schmelzen mit neutralem und saurem pH-Wert angegeben.

In allen Beispielen wurden die folgenden Chemikalien verwendet:

| Calciumlactat | Calcium-Pentahydrat | Fluka 21175 |
| Calciumgluconat | Calcium-D-Gluconat-Monohydrat | Fluka 21142 |
| Calciumacetat | Calciumacetat-Hydrat | Merck 1.09325 |
| Magnesiumchlorid | Magnesiumchlorid-Hexahydrat | Merck 1.05833 |
| Magnesiumlactat | Magnesium-L-Lactact-Hydrat | Fluka 63097 |
| Magnesiumgluconat | Magnesium-D-Gluconat-Hydrat | Fluka 63106 |
| Magnesiumacetat | Magnesiumacetat-Tetrahydrat | Merck 1.05819 |
| Gluconsäure | (50%ig) | Merck 8.22057 |
| Äpfelsäure | | Merck 1.00382 |
| Citronensäure | | Merck 8.18707 |

Beispiel 1

pH-neutrale Schmelze mit Calciumlactat

| 23.16 g | Calciumlactat |
| 16.84 g | Calciumgluconat |

ergibt bei Raumtemperatur eine blanke feste bis duktile Schmelze mit 2.54 Mol Ca/kg Schmelze (entspricht 101 g Ca/kg) mit einem Wasseranteil von ca. 25 % (w/w)

Beispiel 2

neutrale Schmelze mit Calcium-Magnesium-Lactat/Gluconat/Chlorid:

| 10.23 g | Calciumlactat |
| 29.77 g | Calciumgluconat |
| 10.00 g | Magnesiumchlorid |

ergibt bei Raumtemperatur eine blanke feste bis duktile Schmelze mit 1.92 Mol Ca/kg Schmelze (entspricht 76.8 Ca/kg) und 0.95 Mol Magnesium/kg Schmelze (entspricht 23.1 g Magnesium/kg)

Beispiel 3:

pH-neutrale Schmelze mit Calcium und Magnesium im Verhältnis 3:1 (w/w).

| 2.82 g | Calciumlactat |
| 12.29 g | Calciumgluconat |
| 2.89 g | Calciumacetat |
| 0.99 g | Magnesiumlactat |
| 6.05 g | Magnesiumgluconat |
| 1.92 g | Magnesiumacetat |

ergibt bei Raumtemperatur eine blanke, feste bis duktile Schmelze mit 1.67 Mol Ca/kg Schmelze (entspricht 67 g Ca/kg) und 0.83 Mol Magnesium/kg Schmelze (entspricht 21 g Magnesium/kg)

Beispiel 4:

pH-neutrale Schmelze mit Calcium und Magnesium im Verhältnis 3:1 (w/w).

| | |
|---|---|
| 3.25 g | Calciumlactat |
| 9.44 g | Calciumgluconat |
| 5.31 g | Calciumacetat |
| 1.16 g | Magnesiumlactat |
| 4.74 g | Magnesiumgluconat |
| 3.39 g | Magnesiumacetat |

ergibt bei Raumtemperatur eine blanke feste bis duktile Schmelze mit 2.01 Mol Ca/kg Schmelze (entspricht 80 g Ca/kg) und 1.00 Mol Magnesium /kg Schmelze (entspricht 24.3 g Magnesium/kg). Wassergehalt 33 % (w/w).

Beispiel 5:

saure Schmelze mit Calcium, Milchsäure, Äpfelsäure und Gluconsäure.

| | |
|---|---|
| 25.00 g | Calciumlactat |
| 9.42 g | Gluconsäure |
| 1.61 g | Äpfelsäure |

ergibt bei Raumtemperatur eine blanke feste bis duktile Schmelze mit 2.73 Mol Ca/kg Schmelze (entspricht 109 g Ca/kg)

Beispiel 6:

saure Schmelze mit Calcium, Milchsäure, Äpfelsäure, Gluconsäure und Citronensäure.

| | |
|---|---|
| 25.00 g | Calciumlactat |
| 9.42 g | Gluconsäure |
| 1.61 g | Äpfelsäure |
| 2.31 g | Citronensäure |

ergibt bei Raumtemperatur eine blanke feste bis duktile Schmelze mit 2.45 Mol Ca/kg Schmelze (entspricht 98 g Ca/kg). Wassergehalt ca. 27 % (w/w).

Die Reagenzien werden in möglichst wenig Wasser bei Temperaturen um den Siedepunkt gelöst und so weit eingeengt, bis sie die gewünschte Viskosität erlangt haben. Die Verwendung von Vakuum beschleunigt den Vorgang und bringt bessere Ergebnisse.

**Patentansprüche**

1. Verwendung eines Nahrungsmittelzusatzes als konzentriertes Additiv zur Remineralisation von Zahnschmelz, wobei Mineralstoffe in ionisierter Form Bestandteil einer Salzhydratschmelze sind,
   wobei die Mineralstoffe wenigstens Calcium sind,
   wobei die Salzhydratschmelze ein Salz-Wasser-System ist, dessen Wassergehalt der Koordinationszahl des am stärksten hydratisierten Ions entspricht,
   wobei die Salzhydratschmelze amorph erstarrend ist, und
   wobei er bei der Herstellung von Nahrungsmitteln, Genussmitteln, Kaugummi, Getränken, Zahnpasta, Mundwässern, Mundhygieneartikeln oder Zahnpflegemitteln diesen hinzugefügt wird.

2. Verwendung nach Anspruch 1, **dadurch gekennzeichnet, dass** die Mineralstoffe wenigstens ein weiterer Mineralstoff aus der Gruppe Magnesium, Zink, Kalium, Phosphor, Natrium, Selen und Lithium sind.

3. Verwendung nach Anspruch 1 oder 2, **dadurch gekennzeichnet, dass** die Mineralstoffe in der Salzhydratschmelze als Salze von organischen Säuren vorliegen, wobei als Anionen wenigstens einer der Säurereste der organischen Säuren Milchsäure, Gluconsäure, Zitronensäure, Essigsäure, Äpfelsäure, Fumarsäure, Valeriansäure, Ascorbinsäure, Cystein, Glutarsäure oder deren partiell veresterte Abkömmlinge enthalten sind.

4. Verwendung nach einem der Ansprüche 1 bis 3, **dadurch gekennzeichnet, dass** die Mineralstoffe in der Salzhydratschmelze als Salze von anorganischen Säuren vorliegen, wobei als Anionen wenigstens einer der Säurereste der anorganischen Säuren wie Phosphate, Fluoride oder deren partiell veresterte Abkömmlinge enthalten sind.

5. Verwendung nach einem der Ansprüche 1 bis 4, **dadurch gekennzeichnet, dass** der pH-Wert im neutralen Bereich liegt und dass wenigstens eine der Verbindungen Calciumlactat, Calciumgluconat, Calciummalat, Calciumcitrat, Magnesiumlactat, Magnesiumacetat und Magnesiumgluconat enthalten ist.

6. Verwendung nach einem der Ansprüche 1 bis 4, **dadurch gekennzeichnet, dass** der pH-Wert im sauren Bereich liegt und dass wenigstens eine der Säuren Gluconsäure, Äpfelsäure, Milchsäure und Zitronensäure enthalten ist.

7. Verwendung nach einem der Ansprüche 1 bis 6, **dadurch gekennzeichnet, dass** der Salzanteil der Salzhydratschmelze 10 Mol-% bis 25 Mol-% ist.

8. Süßigkeit, umfassend einen Nahrungsmittelzusatz als konzentriertes Additiv zur Remineralisation von Zahnschmelz, wobei Mineralstoffe in ionisierter Form Bestandteil einer Salzhydratschmelze sind, wobei die Mineralstoffe wenigstens Calcium sind, wobei die Salzhydratschmelze ein Salz-Wasser-System ist, dessen Wassergehalt der Koordinationszahl des am stärksten hydratisierten Ions entspricht, wobei die Salzhydratschmelze amorph erstarrend ist, und wobei die Salzhydratschmelze einen im sauren Bereich liegenden pH-Wert aufweist.

9. Süßigkeit nach Anspruch 8, **dadurch gekennzeichnet, dass** die Mineralstoffe wenigstens ein weiterer Mineralstoff aus der Gruppe Magnesium, Zink, Kalium, Phosphor, Natrium, Selen und Lithium sind.

10. Süßigkeit nach Anspruch 8 oder 9, **dadurch gekennzeichnet, dass** die Mineralstoffe in der Salzhydratschmelze als Salze von organischen Säuren vorliegen, wobei als Anionen wenigstens einer der Säurereste der organischen Säuren Milchsäure, Gluconsäure, Zitronensäure, Essigsäure, Äpfelsäure, Fumarsäure, Valeriansäure, Ascorbinsäure, Cystein, Glutarsäure oder deren partiell veresterte Abkömmlinge enthalten sind.

11. Süßigkeit nach einem der Ansprüche 8 bis 10, **dadurch gekennzeichnet, dass** die Mineralstoffe in der Salzhydratschmelze als Salze von anorganischen Säuren vorliegen, wobei als Anionen wenigstens einer der Säurereste der anorganischen Säuren wie Phosphate, Fluoride oder deren partiell veresterte Abkömmlinge enthalten sind.

12. Süßigkeit nach einem der Ansprüche 8 bis 11, **dadurch gekennzeichnet, dass** wenigstens eine der Säuren Gluconsäure, Äpfelsäure, Milchsäure und Zitronensäure enthalten ist.

13. Süßigkeit nach einem der Ansprüche 8 bis 12, **dadurch gekennzeichnet, dass** der Salzanteil der Salzhydratschmelze 10 Mol-% bis 25 Mol-% ist.

14. Süßigkeit nach einem der Ansprüche 8 bis 13, **dadurch gekennzeichnet, dass** sie ausgewählt ist aus Fruchtgummi, Hartkaramell und Kaumasse.

15. Süßigkeit nach einem der Ansprüche 8 bis 14, **dadurch gekennzeichnet, dass** sie Fruchtgummi ist, wobei die Salzhydratschmelze eine saure Mischung aus Calciumgluconat, -lactat, -malat und -citrat enthält.

**Claims**

1. Use of a food additive as a concentrated additive for remineralizing tooth enamel, wherein mineral nutrients are a component of a salt hydrate melt in an ionized form, wherein the mineral nutrients are at least calcium, wherein the salt hydrate melt is a salt-water-system and its water content corresponds to the coordination number of the most hydrated ion, wherein the salt hydrate melt is amorphously solidifying, and wherein it is added in the production of food products, non-essential foods, chewing gum, beverages, toothpaste, mouthwashes, oral hygiene products or tooth care products.

2. Use according to claim 1, **characterized in that** the mineral nutrients are at least one further mineral nutrient from the group of magnesium, zinc, potassium, phosphorus, sodium, selenium and lithium.

3. Use according to claim 1 or 2, **characterized in that** the mineral nutrients are present in the salt hydrate melt as salts of organic acids, wherein at least one of the acid radicals of the organic acids lactic acid, gluconic acid, citric acid, acetic acid, malic acid, fumaric acid, valeric acid, ascorbic acid, cysteine, glutaric acid, or their partially esterified derivatives, is contained therein as anions.

4. Use according to any of claims 1 to 3, **characterized in that** the mineral nutrients are present in the salt hydrate melt as salts of inorganic acids, wherein at least one of the acid radicals of the inorganic acids, such as phosphates, fluorides, or their partially esterified derivatives, is contained therein as anions.

5. Use according to any of claims 1 to 4, **characterized in that** the pH is in the neutral range and that at least one of the compounds calcium lactate, calcium gluconate, calcium malate, calcium citrate, magnesium lactate, magnesium acetate and magnesium gluconate is contained therein.

6. Use according to any of claims 1 to 4, **characterized in that** the pH is in the acidic range and that at least one of the acids gluconic acid, malic acid, lactic acid and citric acid is contained therein.

7. Use according to any of claims 1 to 6, **characterized in that** the salt content of the salt hydrate melt is 10 mol-% to 25 mol-%.

8. Candy, comprising a food additive as a concentrated additive for remineralizing tooth enamel, wherein mineral nutrients are a component of a salt hydrate melt in an ionized form, wherein the mineral nutrients are at least calcium, wherein the salt hydrate melt is a salt-water-system and its water content corresponds to the coordination number of the most hydrated ion, wherein the salt hydrate melt is amorphously solidifying, and wherein the salty hydrate melt has a pH in the acidic range.

9. Candy according to claim 8, **characterized in that** the mineral nutrients are at least one further mineral nutrient from the group of magnesium, zinc, potassium, phosphorus, sodium, selenium and lithium.

10. Candy according to claim 8 or 9, **characterized in that** the mineral nutrients are present in the salt hydrate melt as salts of organic acids, wherein at least one of the acid radicals of the organic acids lactic acid, gluconic acid, citric acid, acetic acid, malic acid, fumaric acid, valeric acid, ascorbic acid, cysteine, glutaric acid, or their partially esterified derivatives, is contained therein as anions.

11. Candy according to one of claims 8 to 10, **characterized in that** the mineral nutrients are present in the salt hydrate melts as salts of inorganic acids, wherein at least one of the acid radicals of the inorganic acids, such as phosphates, fluorides, or their partially esterified derivatives, is contained therein as anions.

12. Candy according to one of claims 8 to 11, **characterized in that** at least one of the acids gluconic acid, malic acid, lactic acid and citric acid is contained therein.

**13.** Candy according to any of claims 8 to 12, **characterized in that** the salt content of the salt hydrate melt is 10 mol-% to 25 mol-%.

**14.** Candy according to any of claims 8 to 13, **characterized in that** it is chosen from fruit-flavored gummi candy, hard candy and chewable compound.

**15.** Candy according to any of claims 8 to 14, **characterized in that** it is fruit-flavored gummi candy, wherein the salt hydrate melt contains an acid mixture of calcium gluconate, calcium lactate, calcium malate and calcium citronate.

## Revendications

**1.** Utilisation d'un complément alimentaire sous forme d'additif concentré pour la reminéralisation de l'émail dentaire, des substances minérales sous forme ionisée étant partie intégrante d'une masse fondue d'hydrates salins, les substances minérales étant au moins du calcium, les masses fondues d'hydrates salins étant un système eau-sel dont la teneur en eau correspond à l'indice de coordination de l'ion le plus fortement hydraté, la masse fondue d'hydrates salins présentant une solidification amorphe, et lors de la fabrication de denrées alimentaires, stimulants, chewing-gums, boissons, dentifrices, eaux dentifrices, articles d'hygiène buccale ou compositions de soins dentaires, le complément alimentaire étant ajouté à ceux-ci.

**2.** Utilisation selon la revendication 1, **caractérisée en ce que** les substances minérales sont au moins une autre substance minérale du groupe magnésium, zinc, potassium, phosphore, sodium, sélénium et lithium.

**3.** Utilisation selon la revendication 1 ou 2, **caractérisée en ce que** les substances minérales dans la masse fondue d'hydrates salins sont présentes sous forme de sels d'acides organiques, au moins l'un des résidus des acides organiques acide lactique, acide gluconique, acide citrique, acide acétique, acide malique, acide fumarique, acide valérique, acide ascorbique, cystéine, acide glutarique ou leurs dérivés partiellement estérifiés étant contenu en tant qu'anion.

**4.** Utilisation selon l'une des revendications 1 à 3, **caractérisé en ce que** les substances minérales dans la masse fondue d'hydrates salins sont présentes sous forme de sels d'acides anorganiques, au moins l'un des résidus des acides anorganiques tels que phosphates, fluorures ou leurs dérivés partiellement estérifiés étant contenu en tant qu'anion.

**5.** Utilisation selon l'une des revendications 1 à 4, **caractérisé en ce que** la valeur pH est située dans la plage neutre et **en ce qu'**est contenu au moins l'un des composés lactate de calcium, gluconat de calcium, malate de calcium, citrate de calcium, lactate de magnésium, acétate de magnésium et gluconat de magnésium.

**6.** Utilisation selon l'une des revendications 1 à 4, **caractérisé en ce que** la valeur pH est située dans la plage acide et **en ce qu'**est contenu au moins l'un des acides acide gluconique, acide malique, acide lactique et acide citrique.

**7.** Utilisation selon l'une des revendications 1 à 6, **caractérisé en ce que** le pourcentage de sel de la masse fondue d'hydrates salins est compris entre 10 Mol-% et 25 Mol-%.

**8.** Friandise, comprenant un complément alimentaire sous forme d'additif concentré pour la reminéralisation de l'émail dentaire, des substances minérales sous forme ionisée étant parties intégrantes d'une masse fondue d'hydrates salins, les substances minérales étant au moins du calcium, la masses fondue d'hydrates salins étant un système eau-sel dont la teneur en eau correspond à l'indice de coordination de l'ion le plus fortement hydraté, la masse fondue d'hydrates salins présentant une solidification amorphe, et la masse fondue d'hydrates salins ayant une valeur pH située dans la plage acide.

**9.** Friandise selon la revendication 8, **caractérisée en ce que** les substances minérales sont au moins une autre substance minérale du groupe magnésium, zinc, potassium, phosphore, sodium, sélénium et lithium.

**10.** Friandise selon la revendication 8 ou 9, **caractérisée en ce que** les substances minérales dans la masse fondue d'hydrates salins sont présentes sous forme de sels d'acides organiques, au moins l'un des résidus des acides organiques acide lactique, acide gluconique, acide citrique, acide acétique, acide malique, acide fumarique, acide valérique, acide ascorbique, cystéine, acide glutarique ou leurs dérivés partiellement estérifiés étant contenu en tant qu'anion.

**11.** Friandise selon l'une des revendications 8 à 10, **caractérisée en ce que** les substances minérales dans la masse fondue d'hydrates salins sont présentes sous forme de sels d'acides anorganiques, au moins l'un des résidus des acides anorganiques tels que phosphates, fluorures ou leurs dérivés partiellement estérifiés étant contenu en tant qu'anion.

**12.** Friandise selon l'une des revendications 8 à 11, **caractérisée en ce qu'**est contenu au moins l'un des acides acide gluconique, acide malique, acide lactique et acide citrique.

**13.** Friandise selon l'une des revendications 8 à 12, **caractérisée en ce que** le pourcentage de sel de la masse fondue d'hydrates salins est compris entre 10 Mol-% et 25 Mol-%.

**14.** Friandise selon l'une des revendications 8 à 13, **caractérisée en ce qu'**elle est choisie parmi gomme aux fruits, caramel dur et pâte à mâcher.

**15.** Friandise selon l'une des revendications 8 à 14, **caractérisée en ce qu'**il s'agit de gomme aux fruits, la masse fondue d'hydrates salins contenant un mélange acide constitué de gluconat, lactate, malate et citrate de calcium.

| Lösungen | | | Schmelzen | | |
|---|---|---|---|---|---|
| Wasser | verdünnte Lösungen | konzentrierte Lösungen | Salzhydrat-schmelzen | hydratisierte Schmelzen | Salz-schmelzen |

$H_2O$–$H_2O$ – WW    Ion–$H_2O$–WW    Ion–Ion–WW

schematisches Zustandsdiagramm des Wechselsystems (WW) Salz-Wasser

0 %    5 %    10 %    25 %    100 %

beispielhafter ungefährer Salzanteil in Prozent

Figur 1

Figur 2a

Figur 2b

**EP 1 916 987 B1**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- DE 10349050 A1
- WO 0044245 A
- EP 0673913 A1
- GB 2341798 A
- US 5851578 A
- GB 1298299 A
- US 4097935 A
- US 4080440 A
- US 4606912 A
- US 5037639 A
- US 5268167 A
- US 5437857 A
- US 5460803 A